# EUROPEAN PATENT SPECIFICATION

(11) **EP 2 113 111 B1**
(45) Date of publication and mention of the grant of the patent: **22.11.2017**
(21) Application number: 08702530.0
(22) Date of filing: 25.01.2008
(51) Int. Cl.: G06F 19/00, H04L 29/08, A61B 5/00

(54) **WIRELESS SENSOR RESIDENT ANNOTATIONS**
RESIDENTE KOMMENTIERUNGEN FÜR EINEN DRAHTLOSEN SENSOR
ANNOTATIONS RÉSIDANT SUR UN CAPTEUR SANS FIL

(30) Priority: 15.02.2007 US 889954 P
(43) Date of publication of application: 04.11.2009
(73) Proprietor: Koninklijke Philips N.V., 5656 AE Eindhoven (NL)
(72) Inventor: ESPINA, Javier, 52064 Aachen (DE); ELIXMANN, Martin, 52074 Aachen (DE)
(74) Representative: de Haan, Poul Erik
(86) International application number: PCT/IB2008/050282
(87) International publication number: WO 2008/099290

(56) References cited:
- WO-A-02/064032
- WO-A-2004/084720
- WO-A-2006/048840
- WO-A-2006/051464
- US-A1- 2002 045 836
- JOVANOV: "Wireless Technology and System Integration in Body Area Networks for m-Health Applications" ENGINEERING IN MEDICINE AND BIOLOGY SOCIETY, 2005. IEEE-EMBS 2005. 27T H ANNUAL INTERNATIONAL CONFERENCE OF THE SHANGHAI, CHINA 01-04 SEPT. 2005, PISCATAWAY, NJ, USA,IEEE, 1 January 2005 (2005-01-01), pages 7158-7160, XP031000876 ISBN: 978-0-7803-8741-6

## Description

The present application relates to patient care. It finds particular application in the transfer of patient responsibility, or hand-off between care givers, but it can also be used in a preclinical or research monitoring setting. More specifically, it relates to reducing medical care errors by improving communication when patient care responsibility is transferred or modified, and will be described with particular attention thereto. It is to be appreciated, however, that the present application is also applicable to any long term care situation, and not only in a hospital patient hand-off setting.

The transfer of patient data and care is commonly referred to as "hand-off." The same healthcare provider does not remain in a healthcare environment all the time, and some patients move between healthcare environments. Different services are provided in different areas of a hospital (e.g. diagnostic imaging, surgery, recovery, and the like). Resultantly, hand-offs are inevitable as a patient progresses through their care regimen. Further, shift changes, break periods, and the like cause changes in healthcare responsibility.

With every hand-off, there comes the potential for breakdowns in communication, which on one end of the spectrum can produce inefficiency, and the same work being done more than once, and on the other end of the spectrum can lead to medical errors. These communication breakdowns can be caused by interruption, omission of information, illegible handwriting, lost information, and the like. Studies suggest that as many as 98,000 people annually die as a result of preventable medical errors. 60% of accidental serious injuries and deaths in healthcare facilities result from breakdowns in communication.

Typically, in-hospital patient monitoring is based on wired connections to sensors that measure the vital parameters of a patient's condition. These measurements are communicated to a patient monitor to be displayed. When a patient moves from place to place, in some environments, old sensors are removed and new ones replace them. In other environments, the sensors are unplugged from a display at the old site and are plugged into a display at the new site. All the cords and wires leading from a patient's sensors can be a nuisance, decreasing the comfort of the patient and requiring creative space saving solutions when space is at a premium, such as in an operating room. In still other environments wireless sensors are used.

Standardized hand off procedures can markedly improve communication, thereby reducing preventable medical errors. One of the most recognized standardized hand-off procedures is the SBAR tool (situation, background, assessment, and recommendation). This particular tool, as well as other standardization procedures, requires the introduction of cards, paper forms, or their electronic equivalent (computers, tablet PCs, PDAs, etc.) into the hospital workflow environment, which adds to overall clutter and decreases accessibility of information. Electronic hand-off information, like its paper counterpart, can be misplaced, lost, or go unnoticed, particularly if a patient is moved.

WO 2004/084720 A2 describes a system for actively monitoring a patient including at least one body-worn monitoring device that has at least one sensor capable of measuring at least one physiologic parameter. At least one intermediary device is linked to the body-worn monitoring device by means of a first wireless network and at least one respondent device is linked to said at least one intermediary device by a second wireless network wherein the respondent device is programmed to perform a specified function automatically when the at least one predetermined event is realized. The monitoring device operates to periodically transmit patient status data to the intermediary device but the system predominantly operates in a quiet state, providing very low power consumption.

WO 2006048840 A1 describes a wireless sensor network for wirelessly monitoring a medical subject including a plurality of sensor nodes. Each sensor node includes a wireless transceiver for sending and receiving wireless messages, a sensor monitoring a characteristic of the medical subject, and a processor. The processor is programmed to at least perform an authentication method including: acquiring sensor data via the sensor for a predetermined time responsive to receiving a wireless trigger message; storing an association code computed from the acquired sensor data; and authenticating a subsequently received wireless message containing an association code tag by comparing the association code tag with the stored association code. The processor further attaches the stored association code as the association code tag in messages sent to other sensors.

The present application provides a new and improved patient monitoring system designed to help eliminate medical errors during patient hand-off that overcomes the above-referenced problems and others.

The object is solved by the features of the independent claims.

Preferably a network is provided. A user uses an input device to create, modify, or delete electronic annotations. A display selectively displays the annotations to a user. A plurality of sensors that are associated with a single patient take measurements of that patient, the sensors including a memories that store data. The network also includes wireless communication devices for wirelessly communicating created annotations, annotation modifications, and annotation deletions to the sensors for storage on at least one of the memories and for communicating annotations wirelessly to at least one of the displays.

In a further preferred embodiment, a method of compiling medical annotations is provided. A plurality of sensors is connected to a subject to form a body sensor network. Annotations are entered. The annotations are wirelessly transmitted to the body sensor network for storage therein. The stored annotations from the body sensor network are wirelessly transmitted to a monitor device for display.

Preferably a method of handing off a patient is provided. Wireless sensor devices are placed in close proximity to a patient. The sensor devices interconnect with each other to form a body sensor network, resulting in all of the sensor devices carrying common data. The patient is moved out of a wireless communication range of a first patient monitor and into the wireless communication range of a second patient monitor, the body sensor network transmitting annotations to the second monitor after the move. The presentation displayed on the patient monitor is changed based on the identity of a healthcare professional accepting the patient hand-off.

In a further preferred embodiment a body sensor network is provided. A plurality of sensors for store annotations pertaining to a subject. A device selectively displays annotations communicated from the sensors based on an annotation category.

One advantage is that the possibility for communication breakdowns during hand-off procedures is reduced.

Another advantage lies in improved versatility.

Another advantage is that it facilitates patient hand off.

Another advantage is that is applicable to a wide variety of monitoring situations.

Another advantage is that annotations remain with a patient.

Another advantage resides in application to research monitoring.

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.
FIGURE 1 is depiction of several typical components of a body sensor network;
FIGURE 2 is a schematic diagram of a body sensor, in accordance with the present application.

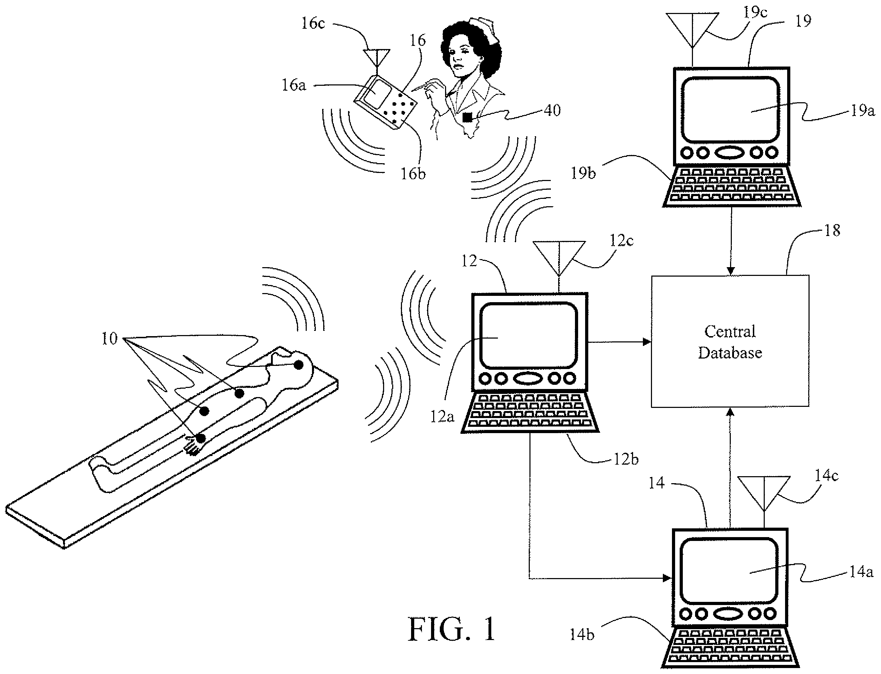

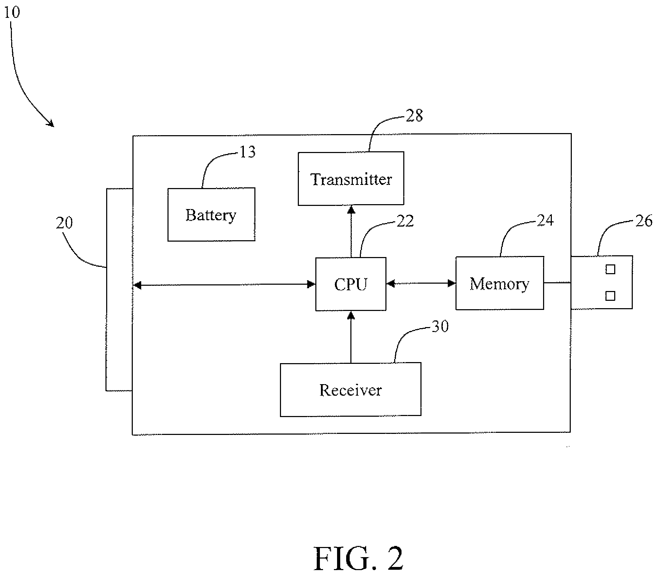

The present application contemplates the use of fully unobtrusive and flexible monitoring of all patients, from non-mobile to mobile patients, and even high acuity patients, such as intensive care unit (ICU) patients. The application also contemplates use in a non-clinical setting, a preclinical setting, and a research setting. Through the use of Body Sensor Networks (BSNs) wireless sensors are used. By using the building blocks of a BSN, a foundation is laid to support the management of electronic annotations. With reference to FIGURE 1, a medical BSN includes a collection of wireless medical sensors **10** that transmit their measurements to a network interface device **12, 14, 16, 19,** such as bedside patient monitor **12** in the vicinity (within, e.g., about 5 meters of the patient), typically associated to a patient display **12a.** The sensors **10** can be nodes of a body network that communicate with each other and the monitor **12** by a short range, low power wireless communication technology such as Bluetooth or ZigBee. Alternately, low power body coupled communication is contemplated for communication between the nodes of the body network, the transmissions to the bedside monitor **12** being performed by a specified node with a larger battery. Each sensor **10** has a battery **13** (shown in FIGURE 2) that powers the functional elements of the sensor **10.** The battery **13** is preferably light weight, and rechargeable, such as a rechargeable lithium ion cell. Other rechargeable batteries and disposable batteries are, of course, contemplated.

The bedside monitor **12** acts as a user interface device for patient related annotations and may be used by a healthcare professional to input, edit, visualize, or delete annotations. An annotation is a piece of information about a given patient. An annotation is typically information input by a physician, nurse, physical therapist, or other healthcare professional concerning a patient's situation, the patient's background, or the patient's assessment and recommendations, particularly recommendations for treatment and care. Typical annotations include medication schedules, bathing schedules, in person visit and motivational schedules, patient's medical conditions, allergies, medicine, interaction warnings, etc. The annotation can be generated anywhere, not necessarily only at the bedside monitor **12,** such as from a physician's office or an electronic device **16** (PDA Tablet PC, Blackberry, etc.) that is connected with the hospital network, a nursing station, **14** or the like.

The sensors **10** periodically or continuously take measurements of a patient's vital signs and these measurements are displayed on the bedside monitor **12** and communicated to the nurses' station and a central database **18** for long term storage. For instance, a blood oxygen monitor continually monitors the patient's pulse, and blood oxygen levels. If the sensors **10** sense a medically significant or threatening event, such as a cardiac arrhythmia, the sensor **10** can automatically generate an annotation reporting the event. It is contemplated that any measurement or monitoring action taken by the sensors **10** can be presented to the healthcare professional in the form of an annotation. That is, the raw data taken by the sensors **10** can be presented as annotations. Another example of an annotation that can be created by a sensor **10** is a "battery low" annotation. If a sensor's battery is low, then it can generate an annotation requesting that the healthcare professional replace or recharge the sensor's **10** battery.

Another example of an event that could trigger annotations could come in association with on-demand pain medication, sometimes prescribed for patients' comfort after particularly rigorous procedures. In this example, when the patient presses a plunger, they receive a measured dose of pain medication. This event may trigger the automatic creation of an annotation, in response to the patient's request. A plurality of pain medication doses could be compiled into a single annotation, such as "the patient requested three doses of pain medication at 1:00 PM, 1:30 PM and 2:00 PM." Annotations taken at the times where the patient is feeling the most pain may help the health care professionals better treat the patient's pain in the future.

Healthcare professionals associated with the patient enter their own annotations, remove old, outdated annotations, denote tasks in annotations as being completed, or the like. Again, this can be done via the bedside monitor **12,** at the nurses' station **14,** from other wireless devices **16,** or from other workstations **19** on the hospital network. Each of the network interface devices **12, 14, 16, 19** has an associated display **12a, 14a, 16a, 19a,** an interface **12b, 14b, 16b, 19b** through which the healthcare professional can enter, modify, delete, etc. the annotations, and a wireless transceiver **12c, 14c, 16c, 19c.** An example of an annotation entered by a healthcare professional could be a record of medications administered. Another example could be a physician's comment about the patient's mental state at a certain time. The possibilities are virtually boundless, but generally, any note that a healthcare professional would want to add to the patient's chart or file can be entered as an annotation. Typical annotations also include to do and have done lists, and other information, which will assist the next care giver to provide a continuity of care without duplications or omissions.

Having described the nature of annotations, the application now moves on to a manner of classification of the annotations. It is contemplated that the annotations will be classified on several parameters, including, but not limited to urgency of the annotation, identity of the person viewing the annotation, age of the annotation, and whether the annotation has previously been viewed.

A first possible category of annotation is "active." An annotation is assigned the active classification if it is new, and has not been viewed by the current attending health care professional yet. When there is one or more active annotations concerning a certain patient, the network interface device **12, 14, 16, 19** can indicate this. Specifically, the display **12a, 14a, 16a, 19a** can automatically display active annotations. It can display an icon indicating that there are active annotations. The network interface device **12, 14, 16, 19** can give an audible chirp if it senses a healthcare professional that has not seen the annotation yet. Sensing the identity of a healthcare professional is discussed more below. Alternately, the network interface device **12, 14, 16, 19** could utilize a combination of any of these notices, as well as others. Other active annotations include lists of activities scheduled for a next shift, such as times to give medications, daily activities, such as a patient bath, which have and have not been performed, unstable physiological parameters that need watching, and the like.

Another category of annotation is "noticed." An active annotation is downgraded to "noticed" after a healthcare professional takes note of the annotation. The healthcare professional can change an active annotation to noticed from the bedside monitor **12,** the nurses' station **14,** or from any other wireless device **16,** or from other input stations **19** associated with the hospital network, as noted above. In one embodiment, the "noticed" annotations are not automatically displayed to the healthcare professional after its initial viewing, but can be recalled by the healthcare professional at will, should the need arise. A healthcare professional may create an annotation and designate it as noticed, for example, when making a note for the next shift. The healthcare professional could create it as noticed and assign a time when it should become active, or it could automatically become active when the monitor **12** senses a new healthcare professional.

Another category of annotation is the "cancelled" annotation. A cancelled annotation is one that is no longer relevant, and will not be displayed to the current healthcare professional, or any later professionals. An example of a cancelled annotation might be a medication that has been dispensed, (although a record of all dispensed medications is retained in the central database **18**), a task that has already been completed, and the like. A cancelled annotation is one that is not likely to be relevant in the present or future. Cancelled annotations can be periodically offloaded to the central database **18** for long term storage. From there they can be accessed at a later date should the need ever arise.

Another possible category of annotation is "critical." These annotations indicate an emergency situation where immediate action by the healthcare professional is required. For example, a pulse sensor might sense that the patient no longer has a pulse. The sensor **10** would generate a critical annotation and possibly trigger an alarm to get a healthcare professional's attention. The sensors can work together in this regard. In another example, the pulse sensor detects no pulse, but an ECG sensor detects a normal heart rhythm. In this case the sensors know that the patient's heart has not stopped, but the BSN may yet create an active annotation requesting the attention of the current healthcare professional, such as "check pulse sensor." Perhaps the pulse sensor has slipped off, or circulation to that area has become poor for some reason. In this example two sensors **10** work together to avoid creating a critical annotation false alarm, but they nonetheless create a lesser degree of annotation.

A more detailed depiction of an exemplary sensor **10** is shown in FIGURE 2. Each sensor **10** has a sensing device **20,** although the body network may have nodes that do not include a sensing device. There are a wide variety of possible sensors **10.** Already mentioned were pulse and blood oxygen sensors, ECG sensors, and blood pressure sensors. Other sensors might include IV flow rate sensors, temperature sensors, brain wave sensors, and the like. A plurality of functions may further be combined into a single sensor. The sensing device **20** is connected to a sensor CPU **22,** which coordinates all of the activities of the sensor **10.** Typically, the CPU directs the sensing device **20** to take a measurement when appropriate, and stores the resultant measurement in an on-board memory **24.** Optionally, the memory can be accessible by a physical connection **26,** such as universal serial bus (USB) connection, IEEE 1394 (firewire) connection, or other similar high-speed data transfer protocol. As mentioned before, the memory **24** can be cleared of cancelled annotations every so often by downloading them to the central database **18** then deleting them from the memory **24.** Typically, the sensors **10** operate independently and create annotations whenever appropriate or requested.

For its wireless communication purposes, the sensor **10** includes a wireless transmitter **28,** e.g. a Bluetooth or a ZigBee transmitter. The sensor **10** uses the wireless transmitter **28** during normal operation, to send its sensor measurements to other sensors or nodes in the network, to the bedside monitor **12,** nurses' station **14,** or other device **16.** Sometimes, such as when a patient is in transport from one area of a hospital to another, a monitor **12** is not within range of the sensors **10.** If this is the case, the sensors continue to store measurements and annotations, if any, and send them as soon as a hospital network interface device **12, 14, 16, 19** is in range.

The sensor **10** also includes a wireless receiver **30,** e.g. a Bluetooth or a ZigBee receiver to receive communications from other sensors **10** or nodes or from the bedside monitor **12.** The sensors **10** can communicate with each other, other nodes, the bedside monitor **12,** nurses' station **14,** other receivers on the hospital network **19** and other electronic devices **16.** Alternately, the sensors communicate with each other and a node by a body coupled communications protocol. The node, such as a wrist band, which carries the body coupled communications transmitter and receiver described above and also a wireless, e.g. Bluetooth or ZigBee transmitter and receiver for communicating with the base station associated with the bedside monitor **12** or other hospital network transmitters and receivers **19,** or medical professional communication devices **16.** The node can also carry a larger battery pack than the other sensors **10** to power the wireless transmissions. Optionally, the node carries the patient ID software to coordinate the adding or removing of sensors from the body network, encryption and decryption software, and the like.

Annotations from medical professionals are transmitted wirelessly to the BSN, either through a designated node or to each sensor individually. The sensors **10** of the BSN are in communication with each other. The sensors **10** routinely send their own readings and annotations to the other sensors **10** in the BSN while receiving readings and annotations from all of the other sensors **10** in the BSN. In one embodiment, all of the sensors **10** within a BSN are updated with the most current annotations concerning the patient. This way, all of the sensors **10** carry the same annotations in their respective resident memories **24.** Thus, if one sensor **10** is removed from the patient's body, malfunctions, or becomes unable to transmit in some other fashion, readings and annotations resident on that sensor's memory **24** are redundantly stored on the other sensors and nodes. This way, as long as at least one sensor **10** remains with the patient (it doesn't matter which one) measurements and annotations concerning that patient will be preserved and readily available.

Following this model, healthcare professionals can swap sensors in and out of the BSN at will, and not all sensors need to be used all the time. For example, perhaps an ultrasound sensor is placed on an expectant mother's abdomen to monitor a fetus's heart rate. Once the baby is born, the sensor is no longer needed, so it can be removed from the patient's body, but the contents of its memory **24** will safely be resident in all of the remaining sensors **10** in the BSN, even if they have not been communicated to a bedside monitor **12,** nurses' station **14,** or other device **16.**

It is preferable that all of the sensors **10** in the BSN store all of the measurements and annotations, so as long as one sensor remains, all of the measurements and annotations remain. Alternately, a master sensor or node that collects and stores the measurements and annotations could be used.

To introduce a sensor **10** into an already existing BSN, the new sensor **10** first must be associated with that BSN. A fresh sensor **10,** such as one that is new, or one that was recently recharging, will not be associated with any BSN. The healthcare professional first associates the fresh sensor with the desired BSN. Each BSN will have a unique identifier, which is then tied back to the identity of the patient. It is to be understood, however, that the identity of the patient does not need to be known. For example, if an unconscious trauma victim is brought to the emergency room by paramedics, but has no identification, the attending staff will assign the patient a unique hospital number and associate the BSN with that number. So even though the patient's identity is not known, the patient's BSN will nevertheless be associated with the patient.

All communications to or from the sensors **10** will include the BSN identifier. This is to prevent cross-talk between BSNs. For example, Patient 1 and Patient 2 each have their own BSN. If the two patients sit down at the same table to have lunch together, there will be no cross-talk between the networks. Since each BSN has its own identifier, Patient 2's sensors will ignore communications from Patient 1's sensors, because they are not associated with the same BSN. The sensors **10** could be actively or passively associated with each other to form the patient's BSN.

Situations may arise where an associated sensor **10** is removed from the BSN or re-associated with a different BSN. In this situation, the redundancy of the BSN keeps data from being lost. Even though the removed sensor **10** is no longer in the BSN, all data carried in its memory will have been already communicated to other sensors in the BSN. So long as there is still at least one sensor associated with the BSN, data from any removed sensors **10** will not be lost. Further, every annotation carries a unique identifier, which identifies, among other things, the BSN (patient) with which the annotation is associated when the annotation was made.

To avoid cluttered communications with the patient monitor **12,** one sensor **10** in the BSN can assume a temporary lead role. For example, if a patient has ten sensors **10** associated with their BSN, it may be undesirable to have all ten sensors **10** trying to communicate with a patient monitor **12.** One of the sensors **10,** perhaps the sensor **10** that has been associated for the longest period of time, could assume a temporary lead role. In this situation, all of the sensors **10** would continue to share annotations with all other sensors **10** in the BSN, but only one would communicate outside the BSN. If the temporary lead sensor **10** is removed from the BSN, no data is lost (again since the sensors **10** do not stop sharing data) but a different sensor **10** assumes the lead role. This might happen after sensing no communications from the previous lead sensor **10** for a period of time.

Once a patient's BSN is running and associated, patient hand-offs can now occur. As mentioned above, a patient hand-off can come in the form of a patient relocation (e.g. from surgery to recovery) or a shift change (e.g. nurse 2 takes over for nurse 1). In both cases, each patient monitor **12** can be associated with a different BSN, or at least be able to distinguish between different BSNs. To illustrate, say a patient is waiting for surgery in a pre-op room. Beside their bed is a monitor **12** associated with their BSN. Another patient monitor **12** awaits in the operating room. When the patient is wheeled from pre-op to the operating room, the pre-op monitor **12** no longer senses the BSN. The operating room monitor **12** senses the patient's BSN when they are wheeled in the room and immediately displays all relevant annotations. Annotations that were classified as noticed can be upgraded to active, because it is assumed that a new healthcare professional or professionals have taken over care of the patient, and those annotations may be relevant and unknown to the new professional(s). Of course, new annotations that were created in the pre-op room or the operating room are also displayed on the operating room monitor **12.**

If it is desirable to have uninterrupted monitoring during patient transport, a BSN enabled measurement server (MMS) can monitor measurements from the wireless sensors in the absence of a patient monitor **12.** For example, the MMS can be detached from a monitor **12** in pre-op and re-attached to an operating room monitor **12.** This would provide uninterrupted monitoring as the patient is in transit.

Another type of hand-off occurs with a shift change, where the patient is not moving, but a new healthcare professional assumes the patient's care. In this case, the new healthcare professional identifies themselves to the patient monitor **12** (or nurses' station **14** or other device **16** and the monitor **12** upgrades noticed annotations to active, to make sure that the new healthcare professional takes note of them. A concept that can assist in this arena is the concept of Active Digital Aura (ADA) which might be based on body coupled communications. The healthcare professional carries with them a wireless ADA transmitter **40** that identifies themselves. This allows the BSNs to automatically detect the identity of the healthcare professional, and allows the BSNs to classify annotations differently for different professionals. Otherwise, the professional would identify themselves, and manually bring up the annotations, such as by pressing a "show all annotations" button. To illustrate, Nurse 1 creates an annotation in the form of a memo that says that Patient has already had the maximum amount of pain medication allowed for the day. The memo is classified as a noticed annotation. When Nurse 1's shift ends, she forgets to tell Nurse 2 that Patient cannot have more pain medication today. But as soon as Nurse 2 walks into Patient's room or comes into close proximity of the patient or the bedside monitor **12,** Nurse 2's ADA is sensed and upgrades the noticed memo to active, since Nurse 2 has not seen it yet. Nurse 2 reads the memo and the information is conveyed even though Nurse 1 forgot to mention it. The concept of Active Digital Aura ensures that as much data as possible is conveyed during a patient hand-off to help prevent medical mistakes.

The nurse, physician, or other healthcare professional can enter annotations on their PDA tablet computer, or other electronic device **16** and wirelessly communicate the annotations to the BSN for storage. Any or all of these devices may be equipped with voice recognition software to record spoken annotations. Other healthcare professionals visiting the patient can read the annotations on their electronic device **16.** Note that these storage and retrieval steps are performed even in the absence of a hospital network. With a hospital network, annotations can be generated at remote locations, such as a healthcare professional's office, communicated over the hospital network to the bedside monitor **12** and wirelessly transmitted to the BSN for storage one or more sensors or nodes. The stored annotations can be retrieved, viewed, modified, and the like using the same path. When using the active digital aura or other healthcare professional identifier, the state (active, noticed, etc.) of each annotation can be differently displayed to each of a plurality of healthcare professionals that are concurrently viewing the same patient's list of annotations in the language of their choice on their viewing device.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims or the equivalents thereof.

## Claims

1. A network comprising:
- an input device (12b, 14b, 16b, 19b) usable for a user to create, modify, or delete electronic annotations;
- one or more displays (12a, 14a, 16a, 19a) adapted to selectively display the annotations to a user;
- a plurality of medical sensors (10) being at least some of a plurality of nodes of a body sensor network, the medical sensors (10) being adapted to be associated with a single patient and to take measurements of that patient, the medical sensors (10) including a memory (24) for storing data, and wherein the medical sensors (10) are adapted to synchronize the data they store so that all of the sensors (10) in the plurality store the same data; and,
- wireless communication devices (12c, 14c, 16c, 19c) adapted to wirelessly communicate created annotations, annotation modifications, and annotation deletions to the medical sensors (10) for storage on the memories (24) and to communicate annotations wirelessly to at least one of the displays.

2. The network as set forth in claim 1, wherein the display (12a, 14a, 16a, 19a) is adapted to display a state of the displayed annotations to the healthcare professional based on the identity of the healthcare professional.

3. The network as set forth in claim 1, wherein the display (12a, 14a, 16a, 19a) is adapted to display a categorized annotation as one of active, noticed, and cancelled.

4. The network as set forth in claim 1, wherein the input device (16b) and the display (16a) are adapted to be associated with a portable electronic device, which is adapted to wirelessly communicate with one or more of the nodes of the body sensor network.

5. The network as set forth in claim 1, further including:
- a medical facility network that includes at least one of the communications devices (12) for communicating wirelessly with the body sensor network, at least one of the input devices (12b, 14b, 16b 19b), at least one display (12a, 14a, 16a, 19a), and a central database (18).

6. The network as set forth in one of claims 1 to 5, wherein
- the plurality of medical sensors (10) are adapted to store common data, and
- annotations communicated from the sensors (10) are selectively displayed on at least one of the displays (12a, 14a, 16a, 19a) based on an annotation category.

7. The network as set forth in claim 6, wherein the annotation category is based at least in part on an identification of a healthcare professional.

8. A method of compiling medical annotations comprising:
- taking measurements of a patient by means of a plurality of medical sensors (10) associated with this single patient, the medical sensors (10) being at least some of a plurality of nodes of a body sensor network;
- receiving created, modified or deleted electronic annotations;
- wirelessly communicating created annotations, annotation modifications or annotation deletions to the medical sensors (10) for storage;
- the medical sensors (10) synchronizing the data they store so that all of the sensors (10) in the plurality store the same data; and
- wirelessly communicating annotations to at least one display (12a, 14a, 16a, 19a) for selectively displaying the annotations to a user.

9. The method as set forth in claim 8 the method further including:
- connecting an additional sensor (10) to the subject; and
- synchronizing the additional sensor (10) with all other sensors (10) in the subject's body sensor network.

10. The method as set forth in claim 8, further including:
wirelessly transmitting annotations from the body sensor network to a portable electronic device (16) for display.

11. The method as set forth in claim 8, further including:
- receiving annotations from a patient monitor (12) that were created at the patient monitor (12).

12. The method as set forth in claim 8, further including designating each annotation as one of active, noticed, and cancelled.

13. The method as set forth in claim 8, further including:
- identifying a healthcare professional viewing an annotation; and,
- changing a designation of an annotation depending on the identity of the healthcare professional viewing the annotation.

14. The method as set forth in claim 8, further including:
- wirelessly transmitting the annotations from the body sensor network to another network.

15. A method of handing off a patient comprising:
- placing a plurality of wireless medical sensor devices (10) for taking measurements of a patient in close proximity to the patient, the medical sensor devices (10) being at least some of a plurality of nodes of a body sensor network;
- allowing the medical sensor devices (10) to interconnect with each other into a body sensor network;
- synchronizing data of all of the medical sensor devices (10) resulting in the medical sensor devices (10) storing the same data;
- moving the patient out of a wireless communication range of a first network interface device (12, 14, 16, 19) and into the wireless communication range of a second network interface device (12, 14, 16, 19), the body sensor network transmitting annotations to the second network interface device (12,14, 16,19) after the move;
- changing a presentation displayed on a display (12a, 14a, 16a, 19a) of the device (12, 14, 16, 19) based on the identity of a healthcare professional accepting the patient hand-off.

## Patentansprüche

1. Netzwerk, umfassend:
- eine Eingabevorrichtung (12b, 14b, 16b, 19b), die von einem Benutzer nutzbar ist, um elektronische Kommentierungen zu erstellen, zu modifizieren oder zu löschen;
- eine oder mehrere Anzeigen (12a, 14a, 16a, 19a), die dafür ausgelegt sind, die Kommentierungen selektiv einem Benutzer anzuzeigen;
- eine Vielzahl von medizinischen Sensoren (10), die zumindest einige von einer Vielzahl von Knoten eines Körpersensornetzwerks sind, wobei die medizinischen Sensoren (10) dafür ausgelegt sind, einem einzelnen Patienten zugeordnet zu werden und Messungen an diesem Patienten durchzuführen, wobei die medizinischen Sensoren (10) einen Speicher (24) zum Speichern von Daten umfassen, und wobei die medizinischen Sensoren (10) dafür ausgelegt sind, die gespeicherten Daten so zu synchronisieren, dass alle Sensoren (10) in der Vielzahl die gleichen Daten speichern; und
- drahtlose Kommunikationsvorrichtungen (12c, 14c, 16c, 19c), die dafür ausgelegt sind, erstellte Kommentierungen, Modifizierungen von Kommentierungen und Löschungen von Kommentierungen drahtlos an die medizinischen Sensoren (10) zur Speicherung in den Speichern (24) zu kommunizieren und Kommentierungen drahtlos an mindestens eine der Anzeigen zu kommunizieren.

2. Netzwerk nach Anspruch 1, wobei die Anzeige (12a, 14a, 16a, 19a) dafür ausgelegt ist, der medizinischen Fachkraft basierend auf der Identität der medizinischen Fachkraft einen Status der angezeigten Kommentierungen anzuzeigen.

3. Netzwerk nach Anspruch 1, wobei die Anzeige (12a, 14a, 16a, 19a) dafür ausgelegt ist, eine kategorisierte Kommentierung als aktiv, bemerkt oder gelöscht anzuzeigen.

4. Netzwerk nach Anspruch 1, wobei die Eingabevorrichtung (16b) und die Anzeige (16a) dafür ausgelegt sind, einer tragbaren elektronischen Vorrichtung zugeordnet zu werden, die dafür ausgelegt ist, mit einem oder mehreren der Knoten des Körpersensornetzwerks drahtlos zu kommunizieren.

5. Netzwerk nach Anspruch 1, weiterhin umfassend:
- ein Netzwerk einer medizinischen Einrichtung, das mindestens eine der Kommunikationsvorrichtungen (12) zum drahtlosen Kommunizieren mit dem Körpersensornetzwerk, mindestens eine von den Eingabevorrichtungen (12b, 14b, 16b, 19b), mindestens eine Anzeige (12a, 14a, 16a, 19a) und eine zentrale Datenbank (18) umfasst.

6. Netzwerk nach einem der Ansprüche 1 bis 5, wobei
- die Vielzahl der medizinischen Sensoren (10) dafür ausgelegt ist, gemeinsame Daten zu speichern, und
- von den Sensoren (10) kommunizierte Kommentierungen selektiv basierend auf einer Kommentierungskategorie auf mindestens einer der Anzeigen (12a, 14a, 16a, 19a) angezeigt werden.

7. Netzwerk nach Anspruch 6, wobei die Kommentierungskategorie zumindest teilweise auf einer Identifizierung einer medizinischen Fachkraft basiert.

8. Verfahren zum Zusammenstellen von medizinischen Kommentierungen, umfassend:
- das Durchführen von Messungen an einem Patienten mit einer Vielzahl von medizinischen Sensoren (10), die diesem einzelnen Patienten zugeordnet sind, wobei die medizinischen Sensoren (10) mindestens einige von einer Vielzahl von Knoten eines Körpersensornetzwerks sind;
- das Empfangen von erstellten, modifizierten oder gelöschten elektronischen Kommentierungen;
- das drahtlose Kommunizieren der erstellten Kommentierungen, der Modifizierungen von Kommentierungen oder der Löschungen von Kommentierungen an die medizinischen Sensoren (10) zur Speicherung;
- wobei die medizinischen Sensoren (10) die gespeicherten Daten so synchronisieren, dass alle Sensoren (10) in der Vielzahl die gleichen Daten speichern; und
- das drahtlose Kommunizieren der Kommentierungen an mindestens eine Anzeige (12a, 14a, 16a, 19a) zum selektiven Anzeigen der Kommentierungen für einen Benutzer.

9. Verfahren nach Anspruch 8, wobei das Verfahren weiterhin Folgendes umfasst:
- das Verbinden eines zusätzlichen Sensors (10) mit dem Subjekt; und
- das Synchronisieren des zusätzlichen Sensors (10) mit allen anderen Sensoren (10) in dem Körpersensornetzwerk des Subjekts.

10. Verfahren nach Anspruch 8, weiterhin umfassend das drahtlose Übertragen von Kommentierungen von dem Körpersensornetzwerk an eine tragbare elektronische Vorrichtung (16) zur Anzeige.

11. Verfahren nach Anspruch 8, weiterhin umfassend:
- das Empfangen von Kommentierungen von einem Patientenmonitor (12), die an dem Patientenmonitor (12) erstellt wurden.

12. Verfahren nach Anspruch 8, weiterhin umfassend:
- das Bezeichnen von jeder Kommentierung als entweder aktiv, bemerkt oder gelöscht.

13. Verfahren nach Anspruch 8, weiterhin umfassend:
- das Identifizieren einer medizinischen Fachkraft, die sich die Kommentierung ansieht; und
- das Ändern einer Bezeichnung einer Kommentierung abhängig von der Identität der medizinischen Fachkraft, die sich die Kommentierung ansieht.

14. Verfahren nach Anspruch 8, weiterhin umfassend:
- das drahtlose Übertragen der Kommentierungen von dem Körpersensornetzwerk an ein anderes Netzwerk.

15. Verfahren zur Übergabe eines Patienten, umfassend:
- das Platzieren einer Vielzahl von drahtlosen medizinischen Sensorvorrichtungen (10) zum Durchführen von Messungen an einem Patienten in unmittelbarer Nähe des Patienten, wobei die medizinischen Sensorvorrichtungen (10) mindestens einige der Vielzahl von Knoten eines Körpersensornetzwerks sind;
- das Erlauben, dass sich die medizinischen Sensorvorrichtungen (10) miteinander zu einem Körpersensornetzwerk verbinden;
- das Synchronisieren der Daten aller medizinischen Sensorvorrichtungen (10), was dazu führt, dass die medizinischen Sensorvorrichtungen (10) die gleichen Daten speichern;
- das Bewegen des Patienten aus einer drahtlosen Kommunikationsreichweite einer ersten Netzwerkschnittstellenvorrichtung (12, 14, 16, 19) heraus und in die drahtlose Kommunikationsreichweite einer zweiten Netzwerkschnittstellenvorrichtung (12, 14, 16, 19) hinein, wobei das Körpersensornetzwerk nach dem Umzug des Patienten Kommentierungen an die zweite Netzwerkschnittstellenvorrichtung (12, 14, 16, 19) überträgt;
- das Ändern einer auf einer Anzeige (12a, 14a, 16a, 19a) der Vorrichtung (12, 14, 16, 19) angezeigten Darstellung basierend auf der Identität einer medizinischen Fachkraft, die die Patientenübergabe entgegennimmt.

## Revendications

1. Réseau comprenant :
- un dispositif d'entrée (12b, 14b, 16b, 19b) utilisable pour qu'un utilisateur crée, modifie ou supprime des annotations électroniques ;
- un ou plusieurs écrans (12a, 14a, 16a, 19a) adaptés pour afficher sélectivement les annotations pour un utilisateur ;
- une pluralité de capteurs médicaux (10) qui sont au moins l'un d'une pluralité de noeuds d'un réseau de capteurs corporels, les capteurs médicaux (10) étant adaptés pour être associés à un patient unique et pour prendre des mesures dudit patient, les capteurs médicaux (10) comportant une mémoire (24) pour stocker des données, et dans lequel les capteurs médicaux (10) sont adaptés pour synchroniser les données qu'ils stockent de sorte que tous les capteurs (10) de la pluralité de capteurs stockent les mêmes données ; et
- des dispositifs de communication sans fil (12c, 14c, 16c, 19c) adaptés pour communiquer sans fil des annotations créées, des modifications d'annotation et des délétions d'annotation aux capteurs médicaux (10) pour le stockage des mémoires (24) et pour communiquer sans fil des annotations à au moins l'un des écrans.

2. Réseau selon la revendication 1, dans lequel l'écran (12a, 14a, 16a, 19a) est adapté pour afficher un état des annotations affichées au professionnel de santé sur la base de l'identité du professionnel de santé.

3. Réseau selon la revendication 1, dans lequel l'écran (12a, 14a, 16a, 19a) est adapté pour afficher une annotation catégorisée en tant qu'annotation active, repérée et supprimée.

4. Réseau selon la revendication 1, dans lequel le dispositif d'entrée (16b) et l'écran (16a) sont adaptés pour être associés à un dispositif électronique portable, qui est adapté pour communiquer sans fil avec l'un ou plusieurs des noeuds du réseau de capteur corporel.

5. Réseau selon la revendication 1, comportant en outre :
- un réseau des installations médicales qui comporte au moins l'un des dispositifs de communication (12) pour communiquer sans fil avec le réseau de capteur corporel, au moins l'un des dispositifs d'entrée (12b, 14b, 16b, 19b), au moins un écran (12a, 14a, 16a, 19a) et une base de données centrale (18).

6. Réseau selon l'une des revendications 1 à 5, dans lequel
- la pluralité des capteurs médicaux (10) est adaptée pour stocker des données communes, et
- les annotations communiquées à partir des capteurs (10) sont sélectivement affichées sur au moins l'un des écrans (12a, 14a, 16a, 19a) sur la base d'une catégorie d'annotation.

7. Réseau selon la revendication 6, dans lequel la catégorie d'annotation est basée au moins en partie sur une identification d'un professionnel de santé.

8. Procédé de collecte d'annotations médicales comprenant :
- la prise de mesures d'un patient au moyen d'une pluralité de capteurs médicaux (10) associés à ce patient individuel, les capteurs médicaux (10) étant au moins certains parmi une pluralité de noeuds d'un réseau de capteurs corporels ;
- la réception des annotations électroniques créées, modifiées ou supprimées ;
- la communication sans fil des annotations créées, des modifications d'annotation ou des suppressions d'annotation aux capteurs médicaux (10) pour stockage ;
- les capteurs médicaux (10) synchronisant les données qu'ils stockent de sorte que tous les capteurs (10) dans la pluralité de capteurs stockent les mêmes données ; et
- la communication sans fil des annotations à au moins un écran (12a, 14a, 16a, 19a) pour afficher sélectivement les annotations à un utilisateur.

9. Procédé selon la revendication 8,
le procédé comportant en outre :
- le raccordement d'un capteur supplémentaire (10) au sujet ; et
- la synchronisation du capteur supplémentaire (10) avec tous les autres capteurs (10) dans le réseau de capteurs corporels d'un sujet.

10. Procédé selon la revendication 8, comportant en outre :
la transmission sans fil d'annotations depuis le réseau de capteurs corporels à un dispositif électronique portable (16) pour affichage.

11. Procédé selon la revendication 8, comportant en outre :
- la réception d'annotations d'un moniteur du patient (12) qui ont été créées sur le moniteur du patient (12).

12. Procédé selon la revendication 8, comportant en outre la désignation de chaque annotation comme étant une annotation active, repérée et supprimée.

13. Procédé selon la revendication 8, comportant en outre :
- l'identification d'un professionnel de santé qui visualise une annotation ; et
- le changement d'une désignation d'une annotation en fonction de l'identité du professionnel de santé qui visualise l'annotation.

14. Procédé selon la revendication 8, comportant en outre :
- la transmission sans fil des annotations du réseau de capteur corporel à un autre réseau.

15. Procédé de transfert d'un patient, comprenant :
- le placement d'une pluralité de dispositifs sans fil de capteurs médicaux (10) pour prendre des mesures d'un patient à proximité immédiate du patient, les dispositifs de capteur médicaux (10) étant au moins certains d'une pluralité de noeuds d'un réseau de capteurs corporels ;
- l'interconnexion des dispositifs de capteurs médicaux (10) les uns avec les autres dans un réseau de capteurs corporels ;
- la synchronisation des données de tous les dispositifs de capteurs médicaux (10) entraînant le stockage des mêmes données par les dispositifs de capteurs médicaux (10) ;
- le déplacement du patient hors d'une sphère de communication sans fil d'un premier dispositif d'interface de réseau (12, 14, 16, 19) et dans la sphère de communication sans fil d'un deuxième dispositif d'interface de réseau (12, 14, 16, 19), le réseau de capteur corporel transmettant des annotations au deuxième dispositif d'interface de réseau (12, 14, 16, 19) après le déplacement ;
- le changement d'une présentation affichée sur un écran (12a, 14a, 16a, 19a) du dispositif (12, 14, 16, 19) sur la base de l'identité d'un professionnel de santé acceptant le transfert de patient.
